# EUROPEAN PATENT APPLICATION

(11) **EP 4 672 252 A1**
(43) Date of publication of application: **31.12.2025**
(21) Application number: 24185127.8
(22) Date of filing: 27.06.2024
(51) Int. Cl.: G16H 10/20, G16H 50/20

(54) **ASSESSING A USER INPUT IN RESPECT TO A SYMPTOM REPRESENTED BY A VIRTUAL PERSON**

(71) Applicant: Siemens Aktiengesellschaft, 80333 München (DE)
(72) Inventor: Johnson, Rebecca, 81739 München (DE)
(74) Representative: Siemens Patent Attorneys

(57) **Abstract**

The disclosure relates to an automated method for assessing a user input in respect to a medical symptom (MS, MS2) represented by a virtual person, the method comprising the steps:
- Configuring (S1) a digital model for a virtual person based on a configuration parameter (CP, MP) by a large langue model (LLM),
the configuration parameter (CP) comprising a medical condition parameter (MP),
- Generating (S2) a visualization (V) of the virtual person based on the digital model,
wherein the virtual person represents a first medical symptom (MS) according to the medical condition parameter (MC),
- receiving (S5) a user input comprising a medical diagnosis (MD),
- assessing (S6) the user input comprising the medical diagnosis (MD) in respect to the medical condition parameter (MP),
and generating (S6) the reply (R) based on a result of the assessment, and
- outputting (S7) the reply (R).

A corresponding system, a computer program product, and a computer-readable storage medium are disclosed as well.

## Description

Independent of the grammatical term usage, individuals with male, female or other gender identities are included within the term.

### DESCRIPTION

### Field of the Invention

The disclosure relates to an automated method for assessing a user input in respect to a medical symptom represented by a virtual person. A corresponding system, a computer program product, and a computer-readable storage medium are disclosed as well.

### Background of the Invention

Physicians must assess patients very effectively in limited time spaces with systematic questions and examinations. Often the illness does not present in bodily symptoms. Therefore, a conversation with the patient is key to finding the right diagnosis and the right treatment.

Currently, conversations with the patient are practised using role play with "actors". This has the disadvantage that the acted situation and the claimed symptoms can come no more than close to the real situation. Another current practise is to practise on real patient. These options are limited, however. A further option for practise is to use written documents describing symptoms or past representations of the illness and use these to practise.

Therefore, conversations with the patient, however, find little practice or focus at universities or in medical education.

Accordingly, there is a need to provide a solution for this need.

### Summary

Embodiments herein generally relate to an automated method for assessing a user input in respect to a medical symptom represented by a virtual person.

The method comprising the step of configuring a digital model for a virtual person based on a configuration parameter (multiple configuration parameter are also possible, the same applies to all other features of the method) by a large langue model. The configuration parameter comprising a medical condition parameter (e.g. a parameter regarding a medical condition, e.g. a medical suffering), e.g. wherein the medical condition parameter is also used for the configuring of the digital model. The virtual person is arranged to for an interaction with a real person. In this step the digital model is generated by the large langue model as backend. The large langue model is also used to amend the digital model for the virtual person during an interaction of the virtual person and the real person.

The method further comprising the step of generating a visualization (e.g. displaying, presenting, outputting the visualisation) of the virtual person based on the digital model (-> based on the configuration parameter and based on the medical condition parameter). The virtual person is generated for interaction with a real person (e.g. a user). The virtual person represents a first medical symptom (a physical symptom, and/or a mental symptom, and/or an injury) according to the medical condition parameter. This step is carried out especially by a 3D computer graphics engine and a displaying unit, e.g. an output device, especially a monitor, and/or glasses (e.g. virtual reality glasses). The generated visualization can be seen as a user interface.

Generating the visualization (e.g. displaying, presenting, outputting the visualisation) of the virtual person is of advantage as it makes the interaction between real person and the virtual person easier to execute. The virtual person is especially generated (displayed) in life-sized. This enables an improved and more realistic interaction between the real person and the virtual person.

The medical condition parameter describes a medical condition. The medical condition leads to a medical symptom (e.g. the first medical symptom and optionally further medical symptoms). This medical symptom (or these symptoms) is shown by the virtual person and presented to the real person through the visualization.

This step optionally is repeated in the way that the visualization is amended (possibly several times) before the next step follows: The (same) virtual person may represent several medical symptoms (see claim 6) before the user input comprising the medical diagnosis is received.

The method further comprising the step of receiving a user input comprising a medical diagnosis (from a real person) especially after the virtual person represented the medical symptom. The real person uses the visualization to practise to make a medical diagnosis by concluding it from the represented first medical symptom. The medical diagnosis is necessary to choose the right treatment. Therefore, in this step the medical diagnosis concluded by the real person is received from the real person via the user input.

Before this step, optionally, a user input comprising a diagnostic question is received (see claim 5). This question is used by the user to receive more details from the virtual person regarding their medical symptoms (see claim 6). These details are used by the real person to make the medical diagnosis (according to this method step).

The method further comprising the step of assessing the user input comprising the medical diagnosis in respect (relation) to the medical condition parameter (e.g. in respect/relation to the represented (first and optional second (further)) medical symptom), and generating the reply based on a result of the assessment. In this step it is assessed if the user input comprises and/or is equal to the right medical condition. If so, the user made the correct medical diagnosis, and the reply contains the information that the right diagnosis was made. If the correct medical diagnose was not yet made, a further user input comprising a medical diagnosis can be received. For the execution of this step (the assessing) the large language model is usable.

The method further comprising the step of outputting the reply. If the reply contains the information that the right diagnosis was made, the training may end afterwards. The training (for the physician) is especially successfully completed when the user (physician) makes the correct diagnosis.

Accordingly, the disclosed method supports a user to practice making a medical diagnosis. The virtual person simulates to be a patient having a certain medical condition. The medical condition is represented to the user by a medical symptom. The user gives a medical diagnose, which they estimate based on the medical symptom. The reply is generated and outputted on the medical diagnosis made by the user.

By involving a prompted large language model combined with a photorealistic virtual person a proper scenario and conversation analogue to a patient-physician reality can be synthesized.

In a further embodiment the virtual person is configured as an avatar. In general, the virtual person is configured to interact with a real person. An avatar is a virtual person or an artificial person in the form of a graphic figure, which represents the embodiment of a person, especially a photorealistic representation. In other words, the virtual person can be represented as a digital person, especially with corresponding body extremities, facial features and the like. In particular, the avatar can also perform movements of the face and body depending on the output signal for the output of the avatar. For example, when the avatar speaks, corresponding mouth movements can be issued. Thus, an extremely realistic communication between the real person and the virtual person, especially the avatar, can be realized.

In a further embodiment the configuration parameter further comprises (e.g. describing and/or parameterizing, e.g. by using labels and/or parameters) a personal age, and/or a personal medical record, and/or a family medical record, and/or a medication plan, and/or a life habit (e.g. eating, drinking, smoking habit), and/or a profession, and/or a location of living, and/or a gender, and/or an ethnicity, and/or a mindset parameter each of the virtual person.

The configuration parameter, especially several configuration parameters, is/are used in the step of configuring the digital model for the virtual person.

Different variables for a configuration parameter allow to configure the virtual person according to different medical conditions and according to different training needs. Further the (general) configuration parameter (compared to the medical condition parameter, which is a species of the configuration parameter) may also influence the medical symptom (represented by the virtual person).

In a further embodiment the medical condition parameter comprises (e.g. describing and/or parameterizing, e.g. by using labels and/or parameters) a medical condition, and/or a timeline on the medical condition, and/or an occurred exposure corresponding to the medical condition, and/or an occurred accident corresponding to the medical condition.

The medical condition parameter comprised by the configuration parameter, especially several medical condition parameters, is/are used in the step of configuring the digital model for the virtual person. Further the first (and optionally the second (further)) medical symptom represented by the virtual person depends on the medical condition parameter.

According to this embodiment, the medical condition is especially comprising (e.g. the medical condition parameter describing and/or parameterizing) a physical condition (e.g. a physical illness/disease), and/or a mental condition (e.g. a physical illness/disease), and/or an injury (after an accident or an event), and/or an infection.

In a further embodiment the method comprises the further step of receiving a user input comprising a diagnostic question (e.g. a question aiming to make a medical diagnosis) (from a real person) especially after the virtual person represented the medical symptom.

The "diagnostic question" is to be understood broad as any statement from the user with the aim to make a diagnosis, this includes assumptions (the virtual person may disagree on) and/or rhetorical questions).

The step of this embodiment is comprised by the method especially before receiving the user input comprising the medical diagnosis.

Further, the step of this embodiment is comprised by the method especially after generating the visualization of the virtual person based on the digital model, wherein the virtual person represents a first medical symptom according to the medical condition parameter.

In a further embodiment the method comprises the further step of generating an amended visualization (e.g. displaying, presenting, outputting the visualisation) of the virtual person based on the digital model (-> based on the configuration parameter and based on the medical condition parameter) and based on the diagnostic question. In the amended visualization of the virtual person represents a second (further) medical symptom (a physical symptom, a mental symptom) according to the medical condition parameter and according to the diagnostic question.

According to this embodiment, the second (further) medical symptom is especially represented by the virtual person in a voice output, and/or a spoken sentence, and/or a description of the medical symptom, and/or a mimic, and/or a gestic, and/or a movement.

The step of this embodiment is comprised by the method especially before receiving the user input comprising the medical diagnosis.

Further, the step of this embodiment is comprised by the method especially after receiving a user input comprising a diagnostic question, according to the previous embodiment. The amended visualization of the virtual person and/or the second (further) medical are to be understood as a reaction and/or an answer from the virtual person on the diagnostic question. The real person may ask (diagnostic question) the virtual person if they also feel a certain way or if they also have another certain symptom. The virtual person reacts on that with representation of the second symptom.

According to this step, in the amended visualization the "same" virtual person is shows. In the amended visualization the virtual person shows a further medical symptom in reaction to the diagnostic question of the real person.

According to this step, the methods suggest a dialogue with several questions from the user and several reactions from the virtual person. Medical conditions, for example, mental illnesses such as dementia or mad cow, that need to be caught by identifying contradicting statements or memory loss can be practiced.

In a further embodiment the virtual person (in the (original) visualization and/or in the amended visualization) further represents a personal mood according to the medical condition parameter.

According to this embodiment, the personal mood is especially represented by the virtual person in a voice output, and/or a spoken sentence, and/or a description of the medical symptom, and/or a mimic, and/or a gestic, and/or a movement.

According to this embodiment, the personal mood of the virtual person is adapted according to the medical condition configured to and suffered by the virtual person. This is a supportive or a leading away factor for the real person to make the medical diagnosis. Further, this makes the interaction with the virtual person more realistic for the user (real person) and the training for the physician more effective.

In a further embodiment the virtual person (in the (original) visualization and/or in the amended visualization) further represents the personal mood further according to the diagnostic question (e.g. a question aiming to make a medical diagnosis) (from a real person).

According to this embodiment, the personal mood is further represented according to the diagnostic question (or any statement of the user). This results in a reaction of virtual person according to actions of real person. The virtual patient may react relieved and/or delighted if physicians shows that they care.

This has the advantage that the real person gets feedback and reactions from the virtual person on their diagnostic questions also via the personal mood of the virtual person. Further, this makes the interaction with the virtual person more realistic for the user (real person) and the training for the physician more effective.

In a further embodiment the first medical symptom is represented by the virtual person in a voice output, and/or a spoken sentence, and/or a description of the medical symptom, and/or a mimic, and/or a gestic, and/or a movement.

According to this embodiment, the virtual person especially describes or shows their symptom (first medical symptom or second medical symptom) through a voice output and/or a spoken sentence, and/or a description of the medical symptom. Mental or psychological symptoms are rather shown (not described) through patterns in spoken sentences. The representation is especially supported by a mimic of the virtual person and a gestic, and/or a movement, like displaying symptoms such as coughing or "knowing" of a rash on his person.

In a further embodiment the user input comprising the medical diagnosis (from a real person) especially after the virtual person represented the medical symptom is received as a voice input and/or a mimic and/or a gestic.

According to this embodiment, the user input comprising the medical diagnosis is especially a spoken sentence or word from the real person, supported by mimic and/or gestic explaining the medical diagnose.

The same applies to the user input comprising the diagnostic question (claim 5): The user input comprising the diagnostic question is received as a voice input, and/or a mimic and/or a gestic. According to this embodiment, the diagnostic question is especially a spoken sentence or word from the real person, supported by mimic and/or gestic explaining the question and/or a further symptom the user might ask for.

In a further embodiment the reply is outputted by a voice output (especially with a corresponding speaking animation (e.g. an animation of mouth and lips of the virtual person), especially plus mimics, gestic) of the virtual person.

The output is the feedback to the real person on their given medical diagnosis. The reply is outputted via the user interface which is also used for displaying the virtual person. "Outputting the reply" is to be understood as making the reply available, e.g. readable and/or hearable and/or visually capturable (e.g. showing a "thumbs up) to the real person (the user).

In a further embodiment the generation of the virtual person for interaction with the real person (e.g. the user) based on the digital model is done by a 3D computer graphics engine (for example by "unreal engine", a series of 3D computer graphics game engines developed by Epic Games) and a displaying unit, e.g. an output device, especially a monitor, and/or glasses (e.g. virtual reality glasses).

Further a system for assessing a user input in respect to a medical symptom represented by a virtual person is disclosed. The system comprising a computational device (electronic computation unit (e.g. a processor)) and an output device (e.g. a displaying unit, e.g. a monitor, and/or glasses (e.g. virtual reality glasses), wherein the system is arranged to execute a method according to this disclosure.

Further a computer program product is disclosed. The computer program product comprising instructions which, when the program is executed by the computational device, cause the computational device to carry out the steps of the method according to this disclosure.

Further a computer-readable storage medium is disclosed. The computer-readable storage medium comprising instructions which, when executed by the computational device, cause the computational device to carry out the steps of the method according to this disclosure.

It is to be understood that the elements and features recited in the appended claims may be combined in different ways to produce new claims that likewise fall within the scope of the present invention. Thus, whereby the dependent claims appended below depend from only a single independent or dependent claim, it is to be understood that these dependent claims can, alternatively, be made to depend in the alternative from any preceding or following claim, whether independent or dependent, and that such new combinations are to be understood as forming a part of the present specification.

While the present invention has been described above by reference to various embodiments, it should be understood that many changes and modifications can be made to the described embodiments. It is therefore intended that the foregoing description be regarded as illustrative rather than limiting, and that it be understood that all equivalents and/or combinations of embodiments are intended to be included in this description.

### Brief Description of the Drawing

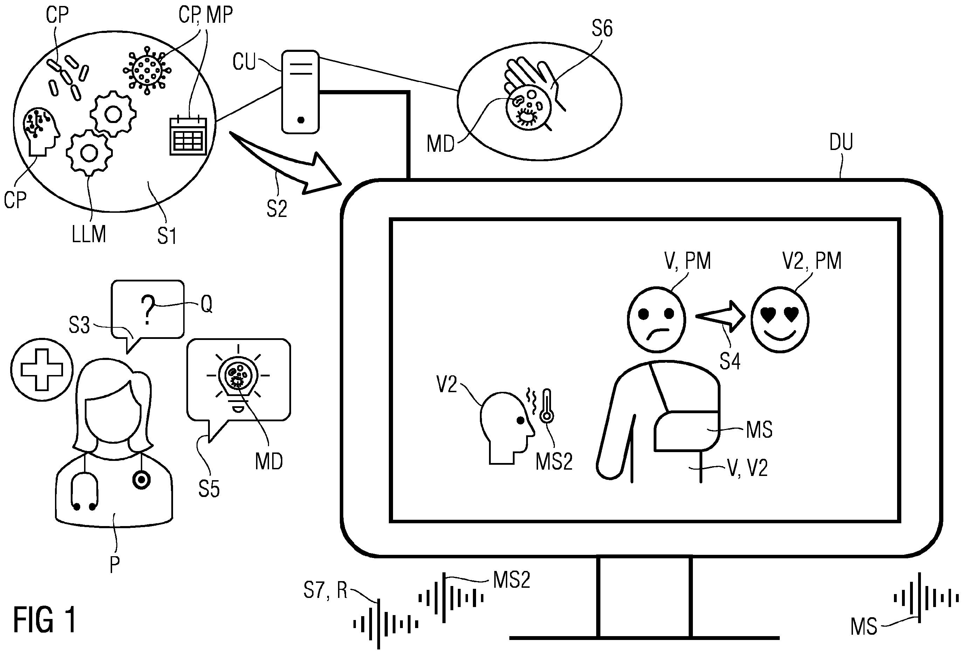

- Fig. 1: shows a system and a corresponding method.

### Detailed Description of the Drawings

Fig. 1 shows a system for assessing a user input in respect to a medical symptom MS, MS2 represented by a virtual person and a corresponding method S1-S7. The system comprising at least one electronic computation unit CU (e.g. a computational device CU, comprising a 3D computer graphics engine) and an output device DU (e.g. displaying unit DU), wherein the system is arranged to execute the corresponding method S1-S7.

The automated method for assessing a user input in respect to a medical symptom MS, MS2 represented by a virtual person, the method comprising the steps:
- Step S1: Configuring a digital model for a virtual person based on a configuration parameter CP, MP by a large langue model LLM. The configuration parameter CP comprising a medical condition parameter MP.
- Step S2: Generating a visualization V of the virtual person based on the digital model, wherein the virtual person represents a first medical symptom MS according to the medical condition parameter MC.
- Step S3: Receiving a user input comprising a diagnostic question Q.
- Step S4: Generating an amended visualization V2 of the virtual person based on the digital model and based on the diagnostic question Q. In the amended visualization V2 of the virtual person represents a second medical symptom MS2 according to the medical condition parameter MC and according to the diagnostic question Q.
- Step S5: Receiving a user input comprising a medical diagnosis MD.
- Step S6: Assessing the user input comprising the medical diagnosis MD in respect to the medical condition parameter MP and generating the reply R based on a result of the assessment.
- Step S7: Outputting the reply R.

In Fig. 1 the configuration parameter CP further comprises a medication plan and a mindset parameter each of the virtual person. The medical condition parameter MP comprises a medical condition and a timeline on the medical condition.

The first medical symptom MS and the second medical symptom MS2 are represented S2 by the virtual person in a voice output MS2 including a spoken sentence and a description of the medical symptom. The representation is supported by a mimic, and/or a gestic, and/or a movement.

The user input comprising the medical diagnosis MD is received S5 as a voice input.

The virtual person further represents a personal mood PM according to the medical condition parameter MP.

Although the invention has been explained in relation to its advantageous embodiment(s) as mentioned above, it is to be understood that many other possible modifications and variations can be made without departing from the scope of the present invention. It is, therefore, contemplated that the appended claim or claims will cover such modifications and variations that fall within the true scope of the invention.

## Claims

1. An automated method for assessing a user input in respect to a medical symptom (MS, MS2) represented by a virtual person, the method comprising the steps:
- Configuring (S1) a digital model for a virtual person based on a configuration parameter (CP, MP) by a large langue model (LLM), the configuration parameter (CP) comprising a medical condition parameter (MP),
- Generating (S2) a visualization (V) of the virtual person based on the digital model, wherein the virtual person represents a first medical symptom (MS) according to the medical condition parameter (MC),
- receiving (S5) a user input comprising a medical diagnosis (MD),
- assessing (S6) the user input comprising the medical diagnosis (MD) in respect to the medical condition parameter (MP),
and generating (S6) the reply (R) based on a result of the assessment, and
- outputting (S7) the reply (R).

2. Method according to claim 1,
wherein the virtual person is configured as an avatar.

3. Method according to one of the previous claims,
wherein the configuration parameter (CP) further comprises:
- a personal age, and/or
- a personal medical record, and/or
- a family medical record, and/or
- a medication plan, and/or
- a life habit, and/or
- a profession, and/or
- a location of living, and/or
- a gender, and/or
- an ethnicity, and/or
- a mindset parameter
each of the virtual person.

4. Method according to one of the previous claims,
wherein the medical condition parameter (MP) comprises:
- a medical condition, and/or
- a timeline on the medical condition, and/or
- an occurred exposure corresponding to the medical condition, and/or
- an occurred accident corresponding to the medical condition.

5. Method according to one of the previous claims,
with the further step of:
- receiving (S3) a user input comprising a diagnostic question (Q).

6. Method according to claim 5,
with the further step of:
- generating (S4) an amended visualization (V2) of the virtual person based on the digital model and based on the diagnostic question (Q),
wherein in the amended visualization (V2) of the virtual person represents a second medical symptom (MS2) according to the medical condition parameter (MC) and according to the diagnostic question (Q).

7. Method according to one of the previous claims,
wherein the virtual person further represents a personal mood (PM) according to the medical condition parameter (MP).

8. Method according to claim 7 and to one of the claims 5 and 6,
wherein the virtual person further represents the personal mood (PM) further according to the diagnostic question (Q).

9. Method according to one of the previous claims,
wherein the first medical symptom (MS) is represented (S2) by the virtual person in:
∘ a voice output, and/or
∘ a spoken sentence, and/or
∘ a description of the medical symptom, and/or
∘ a mimic, and/or
∘ a gestic, and/or
∘ a movement.

10. Method according to one of the previous claims,
wherein the user input comprising the medical diagnosis (MD) is received (S5) as:
- a voice input and/or
- a mimic and/or
- a gestic.

11. Method according to one of the previous claims,
wherein the reply (R) is outputted (S7) by a voice output of the virtual person.

12. Method according to one of the previous claims,
wherein the generation of the virtual person based on the digital model is done by:
- a 3D computer graphics engine (CU), and
- a displaying unit (DU).

13. System for assessing a user input in respect to a medical symptom (MS, MS2) represented by a virtual person,
comprising a computational device (CU) and an output device (DU), wherein the system is arranged to execute a method according to one of the claims 1 to 12.

14. A computer program product comprising instructions which, when the program is executed by a computational device (CU), cause the computational device (CU) to carry out the steps of the method according to one of the claims 1 to 12.

15. A computer-readable storage medium comprising instructions which, when executed by a computational device (CU), cause the computational device (CU) to carry out the steps of the method according to one of the claims 1 to 12.
